# EUROPEAN PATENT SPECIFICATION

(11) **EP 4 334 424 B1**
(45) Date of publication and mention of the grant of the patent: **18.12.2024**
(21) Application number: 22725237.6
(22) Date of filing: 25.04.2022
(51) Int. Cl.: C11D 1/94, C11D 3/04, C11D 3/37, C11D 17/00, A61K 8/36, A61K 8/44, A61K 8/46, A61K 8/86, A61Q 19/10

(54) **HYDRATABLE CONCENTRATED SURFACTANT COMPOSITION SUBSTANTIALLY FREE OF ISETHIONATES**
IM WESENTLICHEN ISETHIONATFREIE HYDRATISIERBARE KONZENTRIERTE TENSIDZUSAMMENSETZUNG
COMPOSITION TENSIOACTIVE CONCENTRÉE HYDRATABLE SENSIBLEMENT EXEMPTE D'ISÉTHIONATES

(30) Priority: 04.05.2021 EP 21171969
(43) Date of publication of application: 13.03.2024
(73) Proprietor: Unilever IP Holdings B.V., 3013 AL Rotterdam (NL); Unilever Global IP Limited, Wirral, Merseyside CH62 4ZD (GB)
(72) Inventor: HIBAN, Douglas John, 6708 WH Wageningen (NL); MOADDEL, Teanoosh, 6708 WH Wageningen (NL); VASUDEVAN, Tirucherai Varahan, 6708 WH Wageningen (NL)
(74) Representative: Fijnvandraat, Arnoldus
(86) International application number: PCT/EP2022/060870
(87) International publication number: WO 2022/233624

(56) References cited:
- WO-A1-2015/179599
- US-A1- 2005 043 194

## Description

### Field of the Invention

The present invention is directed to a hydratable concentrated surfactant composition. The composition is pourable, substantially free of sulfate and oil, comprises a C₆ -C₁₄ acid, amide, alcohol or mixture thereof, anionic surfactant, and an amphoteric surfactant, zwitterionic surfactant or both. The hydratable concentrated surfactant composition further comprises a structuring agent and the anionic surfactant comprises less than 0.0075% by weight isethionate.

### Background of the Invention

Liquid based cleansing compositions, such as shampoos and body washes, are common and enjoyed by many consumers. Such compositions typically have water as the predominant ingredient, and they are often sold in plastic bottles or tubes. The compositions are conventionally formulated to have a viscosity that is customary for consumer use and easy for evacuation from the package they are sold in.

It is often publicized that the world's oceans will soon have more plastic than fish. Given environmental concerns and the desire for consumers and conscious companies to do more for the planet, there is a strong desire to use less plastic when selling products, including consumer products. In view of this, efforts have been made to sell product in concentrate form, and therefore, ship product that comprises less water. The difficulty with concentrates is consumers often do not like adding additional water to the concentrate and further work, like stirring and shaking, to transform the concentrate into an end usable product. As to the hydrated product, common complaints include that the product is not homogeneous after adding water and/or of undesirable viscosity.

It is of increasing interest to develop a concentrate that is easy to pour and hydrate, results in a consumer product that is ready to use in under five (5) minutes and of very desirable characteristics, including viscosity. It is also desirable to develop a concentrate that is substantially free of sulfate and that is easy to use with a refill package to reduce plastic waste. This invention, therefore, is directed to a hydratable concentrated composition that comprises a C₆ -C₁₄ acid, amide, alcohol or mixture thereof, anionic surfactant, and an amphoteric surfactant, zwitterionic surfactant or both. The composition further comprises a structuring agent and the anionic surfactant comprises less than 0.0075% by weight isethionate. The composition is in lamellar phase, and unexpectedly, thickens and transforms to an isotropic phase upon dilution. The composition can be used as a concentrate that is diluted as needed or can be diluted with water in refill packaging to ensure a reduction in plastic waste. Furthermore, the composition of the present invention can be formulated at low pH to better accommodate acidic actives.

### Additional information

Efforts have been disclosed for making wash compositions. In U.S. patent application publication 2019/0314258 A1, rheofluidifying concentrated foaming compositions are described. Further efforts have been disclosed for making wash compositions. In WO2019/074992A1 and WO2019/074993A1, sulfate free personal cleansing compositions with low inorganic salt are described.

Even other efforts have been disclosed for making wash compositions. In U.S. patent application publication 2018/098923 A1, personal care compositions substantially free of sulfated surfactants are described.

Still other efforts have been disclosed for making wash compositions. In U.S. patent application 2019/282480 A1, self-thickening cleansing compositions with N-acyl acidic amino acids or salts thereof and an amphoteric surfactant are described.

US 2005/043194 relates to liquid compositions which thicken on dilution and which comprise electrolyte and associative thickener.

None of the additional information describes surfactant containing compositions as described and claimed in the present invention.

### Summary of the Invention

In a first aspect, the present invention is directed to a lamellar hydratable concentrated surfactant composition having a viscosity from 25 to 10,000 cps (preferably from 25 to 7,500 cps, more preferably, from 250 to 3,500 cps) wherein the composition thickens and increases in viscosity when diluted with water at a composition to water weight ratio from 1:1 to 1:10 (preferably 1:1 to 1:7, more preferably 1:1.5 to 1:6, most preferably 1:1.75 to 1:3) to produce an end use composition having a viscosity from 1,000 to 20,000 cps (preferably 2,000 to 15,000 cps, even more preferably, 3,000 to 12,000 cps, and most preferably, 5,000 to 9,000 cps), the composition comprises from 1 to 30% by weight anionic surfactant wherein less than 0.0075% by weight of the anionic surfactant is isethionate.

In a second aspect, the present invention is directed to the hydratable concentrated surfactant composition of the first aspect of the invention wherein the composition comprises a structurant and transforms from lamellar to isotropic form (i.e., microstructure) upon dilution.

In a third aspect, the present invention is directed to the lamellar hydratable concentrated surfactant composition having a viscosity from 25 to 10,000 cps wherein the composition is suitable to be diluted with water at a composition to water weight ratio from 1:1 to 1:10 to produce an end use composition having a viscosity from 1,000 to 20,000 cps and further wherein the hydratable composition comprises:
a) an anionic surfactant comprising less than 0.0075% by weight acyl isethionate;
b) an amphoteric and/or zwitterionic surfactant;
c) a C₆ -C₁₄ acid, amide and/or alcohol;
d) from 30 to 85% by weight water; and
e) structuring agent,

wherein the anionic surfactant is from 35 to 100% by weight taurate, preferably 100% by weight taurate, and further wherein the composition comprises less than 1 % by weight sulfate, preferably no sulfate, If
wherein the cleansing composition comprises inorganic salt, and If
wherein the pH is from 4.9 to 6.0, and the end use composition having a viscosity that is greater than the viscosity of the hydratable concentrated surfactant composition.

In a fourth aspect, the invention is directed to a hydratable concentrated surfactant composition of the third aspect wherein the structuring agent comprises an ethoxylated fatty acid ester.

In a fifth aspect, the invention is directed to an end use composition made by diluting at least one of the hydratable concentrated surfactant compositions of the first four aspects of the invention wherein the end use composition produced has a viscosity that is greater than the viscosity of the hydratable concentrated surfactant composition it is prepared from.

In a sixth aspect, the invention is directed to the use of the end use composition of the fifth aspect of the invention to cosmetically treat skin by cleaning the skin.

As used herein, "compositions" with no qualifier is meant to mean the hydratable concentrated surfactant composition and end use composition of this invention. Hydratable, as used herein, means add and/or add and absorb water (i.e., to dilute) even to a composition that has water such as a composition that is initially 30 to 85% by weight water. Skin, as used herein, is meant to include skin on the arms (including underarms), face, feet, neck, chest, hands, legs, buttocks and scalp (including hair). Hydratable concentrated surfactant composition ("hydratable composition") means a lamellar composition that increases in viscosity when water is added to the composition to thereby produce an isotropic end use composition suitable for topical application. Lamellar, as used herein, means a two-dimensional phase with lipid bilayers separated by water layers, an opaque, hazy and/or cloudy composition having a birefringent pattern when viewed in an optical microscope. Isotropic means having lipid layers of one dimension and a transparent composition that does not display a birefringent pattern when viewed in an optical microscope. Transforming from a lamellar to an isotropic phase is also confirmed by visual examination of the concentrate becoming a transparent end use composition after the hydratable composition is hydrated. The hydratable composition is one which is suitable to optionally have a viscosity from 500 to 1,500 cps. (1 Pa-s is equal to 1000 cps). The end use composition is one suitable to be wiped or washed off, and preferably, washed off with water. The end use composition can be a home care cleaning composition but is preferably a shampoo, make-up wash, facial wash, hand wash or personal care liquid body wash. In an embodiment of the invention, the end use composition can optionally have a viscosity from 6,000 to 12,000 cps when a body wash and from 2,000 to 5,000 cps when a hand wash. The end use composition may, optionally, comprise medicinal or therapeutic agents, but preferably, is a wash which is cosmetic and non-therapeutic such that the wash removes water soluble and water insoluble soils. In one embodiment of the invention, the end use composition is a home care composition like a table-top or toilet cleaning composition. In another embodiment, the end use composition is a shampoo composition. In still another embodiment, the end use composition is a personal wash composition, and therefore, a liquid body wash. As hereinafter described, the end use composition of the present invention may optionally comprise skin benefit ingredients added thereto such as emollients, vitamins and/or derivatives thereof, resorcinols, retinoic acid precursors, colorants, moisturizers, sunscreens, mixtures thereof or the like. The skin benefit ingredients (or agents) may be water or oil soluble. If used, oil soluble skin benefit agents typically make up to 1.5% by weight of the hydratable composition whereby water soluble skin benefit agents, when used, typically make up to 10% by weight of the hydratable composition of the present invention. The hydratable composition and end use composition typically have a pH from 3.5 to 10. Viscosity, unless noted otherwise, is taken with a Discovery HR-2 Rheometer using sand blasted plates with a 100 micron gap and a shear rate of 4-15 s⁻¹. Viscosity is measured at 25 °C. Increase in viscosity means the hydratable composition of the present invention will have a starting viscosity that is lower than the final viscosity after water is added and the resulting end use composition is made. The end use composition is made by combining water and hydratable composition and mixing (with moderate shear like stirring, preferably shaking) the same to produce the end use composition having a higher viscosity than the hydratable concentrate it is made from. In another embodiment, the hydratable composition may be applied directly to, for example, skin of a consumer and when water and shear are applied (like, for example, shearing with the hand and water from a sink or shower) the desired end use composition may be made. As used herein, "substantially free of sulfate" means less than 6.0% by weight of the end use composition, and "substantially free of oil" means less than 0.3% by weight of the end use composition. Substantially free of isethionate means the anionic surfactant has less than 0.0075% by weight isethionate, and preferably, less than 0.0050% by weight isethionate, and most preferably, 0.0% by weight (i.e., no isethionate) based on total weight of the anionic surfactant in the hydratable composition. Structuring agent means suitable to build viscosity in a hydrated composition substantially free of isethionate. Lamellar phase agent means the agent that induces lipid bilayer formation separated by water layers. The term comprising is meant to encompass the terms consisting essentially of and consisting of. For the avoidance of doubt, and for illustration, the end use composition of this invention comprising surfactant, water and active is meant to include a composition consisting essentially of the same and a composition consisting of the same. All ranges defined are meant to include all ranges subsumed therein. Except in the operating comparative examples, or where otherwise explicitly indicated, all numbers in this description indicating amounts or ratios of materials or conditions and/or physical properties of materials and/or use are to be understood as modified by the word "about".

### Detailed Description of the Invention

As to the anionic surfactant, the same typically makes up from 1.0 to 30% by weight of the hydratable composition. In an embodiment of the invention, the anionic surfactant makes up from 2.0 to 25% by weight, and preferably, from to 3.0 to 20% by weight, and most preferably, from 6 to 15% by weight of the hydratable composition. Still in another embodiment, anionic surfactant makes up from 7 to 14% by weight of the hydratable composition. The anionic surfactant is from 35 to 100% by weight taurate based on total weight of anionic surfactant used in the compositions. Glycinate may also be present.

As to the amphoteric and/or zwitterionic surfactant used in the hydratable composition, the same typically makes up from 1.0 to 45%, and preferably, from 2.0 to 35%, and most preferably, from 12 to 25% by weight of the hydratable composition.

To, for example, aid in hydratable composition lamellar phase stabilization and hydration, a lamellar phase agent like C₆ -C₁₄ acid, amide and/or alcohol is preferably used and typically makes up from 1.0 to 16%, and preferably, from 1.8 to 12%, and most preferably, from 3 to 8% by weight of the hydratable concentrated composition. The preferred agent for use is myristic acid, lauric acid and any alcohol or amide derivatives thereof. In a most preferred embodiment, the lamellar phase agent used is lauric acid. In yet another preferred embodiment, the lamellar phase agent makes up from 4.2 to 5.2% by weight of the hydratable concentrated composition.

Inorganic salt aids in composition stabilization. Typical salts may be used like NaCl, KCI, MgCl₂, CaCl₂, mixtures thereof or the like. Typically, the inorganic salt makes up to 15%, and preferably, from 1 to 12%, and most preferably from 0.75 to 4.5% by weight of the hydratable concentrated composition. In an embodiment of the invention, salt makes up from 2.5 to 3.5% by weight of the hydratable concentrated composition.

Structurants, often polymeric viscosity aids, are an ingredient in the hydratable composition of the present invention. Illustrative examples of the structurants suitable for use in the invention include esters of polyalkoxylated polyols and fatty acids. Examples of such structurants include PEG 18 glyceryloleate/cocoate, polyethylene glycol 6000 distearate, INCI name of PEG-150 distearate; PEG 120 methyl glucose dioleate and PEG 120 methylglucose trioleate (Glucomate^{™} DOE-120 and Glucomate^{™} VLT made available by Lubrizol); PEG-150 Pentaerythrityl Tetrastearate (Crothix^{™}, Crothix^{™} Liquid, and Versathix^{™} made available by Croda); PEG-150 Polyglyceryl-2 Tristerate (Genapol^{®} LT made available by Clariant); PEG/PPG-120/10-Trimethlolpropane Trioleate (Arlypon^{®} TT made available by BASF). The number of hydrophilic polyalkoxylated arms are two for PEG-150 distearate, three for Arlypon^{®} TT, four for Genapol^{®} LT and Crothix^{™}, Crothix^{™} Liquid, and Versathix^{™}, and five for Glucomate^{™} DOE-120.

The preferred structurants (classified as high molecular weight ethoxylated fatty acid esters) are PEG 120 methyl glucose dioleate, as well as PEG 150 pentaerythrityl tetrastearate. The structurants used ideally make up from 0.1 to 1.2%, and preferably, from 0.2 to 1.0%, and most preferably, from 0.25 to 0.8% by weight of the hydratable composition. In an embodiment of the invention, the structurant makes up from 0.35 to 0.65% by weight of the hydratable composition. In the invention, less than 1.0% by weight sulfate is present in the end use composition of the present invention, preferably no (0.0% by weight) sulfate. In the present invention, the hydratable composition should be formulated such that upon dilution, the desired component/ingredient levels (such as sulfate levels) in the end use composition are attained.

As to anionic surfactants suitable for use in the hydratable concentrated surfactant composition and end use composition of the present invention, the anionic surfactant used can include aliphatic sulfonates, such as a primary alkane (e.g., C₈-C₂₂) sulfonate, primary alkane (e.g., Cs-C₂₂) disulfonate, C₈-C₂₂ alkene sulfonate, C₈-C₂₂ hydroxyalkane sulfonate or alkyl glyceryl ether sulfonate (AGS); or aromatic sulfonates such as alkyl benzene sulfonate. The anionic can also include some alkyl sulfate (e.g., C₁₂-C₁₈ alkyl sulfate) or alkyl ether sulfate (including alkyl glyceryl ether sulfates). Among the alkyl ether sulfates are those having the formula: RO(CH₂CH₂O)ₙSO₃M
wherein R is an alkyl or alkenyl having 8 to 18 carbons, preferably 12 to 18 carbons, n has an average value of at least 1.0, preferably less than 5, and most preferably 1 to 4, and M is a solubilizing cation such as sodium, potassium, ammonium or substituted ammonium. As noted, sulfates, when used, make up less than 6.0% by weight of the end use composition.

The anionic may also include alkyl sulfosuccinates (including mono- and dialkyl, e.g., C₆-C₂₂ sulfosuccinates); alkyl and acyl sarcosinates sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, sulphoacetates, alkyl glucosides and acyl isethionates, and the like.

Sulfosuccinates may be monoalkyl sulfosuccinates having the formula:

R¹O₂CCH₂CH(SO₃M)CO₂M;

and amide-MEA sulfosuccinates of the formula:

R¹CONHCH₂CH₂O₂CCH₂CH(SO₃M)CO₂M wherein R¹ ranges from C₈-C₂₂ alkyl.

Sarcosinates are generally indicated by the formula:

R²CON(CH₃)CH₂CO₂M, wherein R² ranges from C₈-C₂₀ alkyl.

Taurates are generally identified by formula:

R³CONR⁴CH₂CH₂SO₃M

wherein R³ is a C₈-C₂₀ alkyl, R⁴ is a C₁-C₄ alkyl.

M is a solubilizing cation as previously described.

Less than 0.0075% weight of the total weight of anionic used in the hydratable composition should be isethionate. If used, the isethionates may include C₈-C₁₈ acyl isethionates (including those which have a substituted head group such as a C₁₋₄ alkyl substitution, preferably methyl substitution). These esters are prepared by a reaction between alkali metal isethionate with mixed aliphatic fatty acids having from 6 to 18 carbon atoms and an iodine value of less than 20. Often at least 75% of the mixed fatty acids have from 12 to 18 carbon atoms and up to 25% have from 6 to 10 carbon atoms.

The small amount of acyl isethionate that may optionally be used can be an alkoxylated isethionate such as is described in Ilardi et al., U.S. Pat. No. 5,393,466, entitled "Fatty Acid Esters of Polyalkoxylated isethonic acid; issued Feb. 28, 1995.

This compound has the general formula:

R⁵C--O(O)--C(X)H--C(Y)H--(OCH₂--CH₂)ₘ--SO₃M

wherein R⁵ is an alkyl group having 8 to 18 carbons, m is an integer from 1 to 4, X and Y are each independently hydrogen or an alkyl group having 1 to 4 carbons and M is a solubilizing cation as previously described. Preferably, the hydratable concentrated composition is substantially free of isethionate.

In an embodiment of the invention, the anionic surfactant used is sodium lauroyl glycinate, sodium cocoyl glycinate, sodium lauroyl glutamate, sodium cocoyl glutamate, sodium methyl lauroyl taurate, sodium methyl cocoyl taurate or a mixture thereof. Such anionic surfactants are commercially available from suppliers like Galaxy Surfactants, Clariant, Sino Lion and Innospec. Sodium methyl lauroyl taurate, sodium lauroyl glycinate or mixtures thereof are the preferred anionics used in this invention.

Amphoteric surfactants suitable for use in the invention (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants suitable for use include sodium lauroamphoacetate, sodium cocoamphoacetate and mixtures thereof. If present such surfactants make up from 0.01 to 5% and preferably, from 0.1 to 2.5% by weight of the hydratable composition.

As to the zwitterionic surfactants that may be employed in the present invention, such surfactants include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms generally comply with an overall structural formula:
R⁶--[--C(O)--NH(CH₂)_{q}--]ᵣ--N⁺--(R⁷--)(R⁸)A--B where R⁶ is alkyl or alkenyl of 7 to 18 carbon atoms; R⁷ and R⁸ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is --CO₂-- or --SO₃--.

Suitable zwitterionic surfactants for use in the present invention and within the above general formula include simple betaines of formula:

R⁶-N⁺-(R⁷)(R⁸)CH₂CO₂⁻

and amido betaines of formula:

R⁶-CONH(CH₂)ₜ-N⁺-(R⁷)(R⁸)CH₂CO₂⁻where t is 2 or 3.

In both formulae R⁶, R⁷ and R⁸ are as defined previously. R⁶ may, in particular, be a mixture of C₁₂ and C₁₄ alkyl groups derived from coconut oil so that at least half, preferably at least three quarters of the groups R⁶ have 10 to 14 carbon atoms. R⁷ and R⁸ are preferably methyl.

A further possibility is that the zwitterionic surfactant is a sulphobetaine of the formula:

R⁶--N⁺--(R⁷)(R⁸)(CH₂)₃SO₃⁻

or

R⁶--CONH(CH₂)ᵤ --N⁺-(R⁷)(R⁸)(CH₂)₃SO₃⁻

where u is 2 or 3, or variants of these in which --(CH₂)₃SO₃⁻ is replaced by-CH₂C(OH)(H)CH₂SO₃⁻.

In these formulae, R⁶, R⁷ and R⁸ are as previously defined.

Illustrative examples of the zwitterionic surfactants suitable for use include betaines like cocodimethyl carboxymethyl betaine, cocamidopropyl betaine and laurylamidopropyl betaine. An additional zwitterionic surfactant suitable for use includes cocamidopropyl sultaine. Such surfactants are made commercially available from suppliers like Stepan Company, and it is within the scope of the invention to employ mixtures of the aforementioned surfactants. In a preferred embodiment, the zwitterionic surfactant used in the compositions of this invention is cocamidopropyl betaine.

Zwitterionic surfactants are preferably used and make up from 2 to 28%, and preferably, from 6 to 25%, and most preferably, from 12 to 22% by weight of the hydratable composition. In an embodiment of the invention, zwitterionic surfactant makes up from 16 to 21% by weight of the hydratable concentrate.

Nonionic surfactants may optionally be used in the hydratable composition and end use composition of the present invention. When used, nonionic surfactants are typically used at levels as low as 0.5, 1, 1.5 or 2% by weight and at levels as high as 6, 8, 10 or 12% by weight of the end use composition. The nonionics which may be used include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like.

In an embodiment of the invention, nonionic surfactants optionally used can include fatty acid/alcohol ethoxylates having the following structures a) HOCH₂(CH₂)ₛ(CH₂CH₂O)ᵥ H or b) HOOC(CH₂)_{c}(CH₂CH₂O)_{d} H; where s and v are each independently an integer up to18; and c and d are each independently an integer from 1 or greater. In an embodiment of the invention, s and v are each independently 6 to 18; c and d are each independently 1 to 30. Other options for nonionic surfactants include those having the formula HOOC(CH₂)ᵢ-CH=CH-(CH₂)ₖ(CH₂CH₂O)_{z} H, where i, k are each independently 5 to 15; and z is 5 to 50. In another embodiment of the invention, i and k are each independently 6 to 12; and z is 15 to 35.

The nonionic may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991.

In an embodiment of the invention, cationic surfactants may optionally be used in the hydratable composition and end use composition of the present invention.

One class of optional cationic surfactants includes heterocyclic ammonium salts such as cetyl or stearyl pyridinium chloride, alkyl amidoethyl pyrrylinodium methyl sulfate, and lapyrium chloride.

Tetra alkyl ammonium salts are another useful class of cationic surfactants suitable for optional use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and strearyl amidopropyl dimethylamine lactate.

Even other useful cationic surfactants suitable for optional use include quaternized hydrolysates of silk, wheat, and keratin proteins, and it is within the scope of the invention to use mixtures of the aforementioned cationic surfactants.

If used, cationic surfactants will make up no more than 1.0% by weight of the hydratable composition. When present, they typically make up from 0.01 to 0.7%, and more typically, from 0.1 to 0.5% by weight of the end use composition, including all ranges subsumed therein.

In an embodiment of this invention, the end use composition of this invention will be substantially free of polymeric quaternary ammonium compounds (including salts of the same). In another embodiment, the end use composition will comprise less than 0.1% by weight polymeric quaternary ammonium compounds. In yet another embodiment, the end use composition comprises less than 0.01% by weight polymeric quaternary ammonium compounds. In even another embodiment, the hydratable composition and end use composition are free of polymeric quaternary ammonium compounds (i.e., 0.0%).

Water makes up from 30 to 85%, typically, from 30 to 78%, and preferably, from 35 to 75% by weight of the hydratable composition, and most preferably, from 40 to 70% by weight of the hydratable composition.

The pH of the hydratable composition is from 4.9 to 6.

Optional skin benefit agents suitable for use in this invention are limited only to the extent that they are capable of being topically applied, and suitable to dissolve in the hydratable composition and end use composition at the desired pH.

Illustrative examples of the benefit agents suitable to include in the water portion of the compositions are vitamin B₂, niacinamide (vitamin B₃), vitamin B₆, vitamin C, mixtures thereof or the like. Water soluble derivatives of such vitamins may also be employed. For instance, vitamin C derivatives such as ascorbyl tetraisopalmitate, magnesium ascorbyl phosphate and ascorbyl glycoside may be used alone or in combination with each other. Other water soluble benefit agents suitable for use include 4-ethyl resorcinol, extracts like sage, aloe vera, green tea, grapeseed, thyme, chamomile, yarrow, cucumber, liquorice, rosemary extract or mixtures thereof. Water soluble sunscreens like ensulizole may also be used. Total amount of optional water soluble benefit agents (including mixtures) when present in the invention may range from 0.0 to 10%, preferably from 0.001 to 8%, and most preferably, from 0.01 to 6% by weight, based on total weight of the end use composition.

It is also within the scope of the present invention to optionally include oil (i.e., non-water) soluble benefit agents. The end use composition is substantially free of oil, and preferably, has less than 0.15% by weight oil, and most preferably, no oil (0.0%) where oil is not meant to include any oil from a fragrance. Thus, oil soluble actives or benefit agents are solubilized in the surfactants used. The only limitation with respect to such oil soluble benefit agents are that the same are suitable to provide a benefit when topically applied. The composition has preferably less than 0.15% petrolatum (petroleum jelly), more preferably is free from petrolatum.

Illustrative examples of the types of oil soluble benefit agents that may optionally be used in the compositions of this invention include components like stearic acid, vitamins like Vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or mixtures thereof.

Other optional oil soluble benefit agents suitable for use include resorcinols like 4-hexyl resorcinol, 4-phenylethyl resorcinol, 4-cyclopentyl resorcinol, 4-cyclohexyl resorcinol 4-isopropyl resorcinol or a mixture thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Published Patent Application No. 2016/0000669A1.

Even other oil soluble actives suitable for use include omega-3 fatty acids, omega-6 fatty acids, climbazole, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, terpineol, thymol mixtures thereof or the like.

In an embodiment of the invention, the optional oil soluble benefit agent used is a retinoic acid precursor. In one embodiment of the invention, the retinoic acid precursor is retinol, retinal, retinyl propionate, retinyl palmitate, retinyl acetate or a mixture thereof. Retinyl propionate, retinyl palmitate and mixtures thereof are typically preferred. Additionally, 12-hydroxystearic acid is often preferred for use.

When optional oil soluble active is used in the compositions of the invention, it typically makes up from 0.0 to 1.5%, and preferably, from 0.001 to 1.5%, and most preferably, from 0.05 to 1.2% by weight of the end use composition. In yet another embodiment, oil makes up from 0.1 to 0.5% by weight of the total weight of the end use composition.

Preservatives can desirably be incorporated into the hydratable concentrated surfactant composition and end use composition to protect against the growth of potentially harmful microorganisms. Cosmetic chemists are familiar with appropriate preservatives and routinely choose them to satisfy the preservative challenge test and to provide product stability. Suitable traditional preservatives for use include hydantoin derivatives and propionate salts. Particularly preferred preservatives are iodopropynyl butyl carbamate, phenoxyethanol, sodium benzoate, hydroxyacetophenone, ethylhexylglycerine, hexylene glycol, methyl paraben, propyl paraben, imidazolidinyl urea, sodium dehydroacetate, dimethyl-dimethyl (DMDM) hydantoin and benzyl alcohol and mixtures thereof. Other preservatives suitable for use include sodium dehydroacetate, chlorophenesin and decylene glycol. The preservatives should be selected having regard for the use of the composition and possible incompatibilities between the preservatives and other ingredients in the emulsion. Preservatives are preferably employed in amounts ranging from 0.01% to 2.0% by weight of the total weight of the end use composition (up to 7% by weight of total hydratable concentrated surfactant composition), including all ranges subsumed therein. Also preferred is a preservative system with hydroxyacetophenone alone or in a mixture with other preservatives. Standard emollients, like vicinal diols (e.g., 1,2-hexane diol and/or 1,2-octane diol) may be used with the preservatives. In an embodiment of the invention, the preservative used is sodium benzoate when the pH of the compositions is 7 or under, and preferably, 6 or under.

Thickening agents are optionally suitable for use in the compositions of the present invention.

Particularly useful are the polysaccharides. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose). Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

Synthetic polymers, in addition to structurants, are yet another class of thickening agents that can optionally be used. This category includes crosslinked polyacrylates such as the Carbomers, polyacrylamides such as Sepigel^{®} 305 and taurate copolymers such as Simulgel^{®} EG and Aristoflex^{®} AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used.

The amounts of optional thickening agent, when used, may range from 0.001 to 5%, by weight of the compositions. Maltodextrin, xanthan gum, and carboxymethyl cellulose are the often preferred optional thickening agents.

In an embodiment of the invention and given the compositions of the present invention are substantially free of isethionates, the pH of the compositions are in the acidic range, and the desired preservative is sodium benzoate and the end use composition is formulated with from 0.01 to 4%, and preferably, from 0.05 to 1.5%, and most preferably, from 0.05 to 0.6% by weight of an acidic active or skin benefit agent. Often preferred acidic actives include alpha hydroxy acids like citric acid, glycolic acid, lactic acid, malic acid, tartaric acid or mixtures thereof. Beta hydroxy acids are also suitable for use and these include salicylic acid, salicylate, sodium salicylate, willow, beta hydroxybutanoic acid, tropic acid trethocanic acid or mixtures thereof. Amino acids are also desirable for use and these include arginine, valine and/or histidine.

Fragrances, fixatives, chelators (like EDTA) and exfoliants may optionally be included in the compositions of the present invention. Each of these substances may range from about 0.03 to about 5%, preferably between 0.1 and 3% by weight of the total weight of the end use composition, including all ranges subsumed therein. To the extent the exfoliants are used, those selected should be of small enough particle size so that they do not impede the performance of any packaging used to dispense the compositions of this invention.

Conventional emulsifiers having an HLB of greater than 8 may optionally be used. Illustrative examples include Tween, 40, 60, 80, polysorbate 20 and mixtures thereof. Typically, emulsifiers for water continuous systems make up from 0.3 to 2.5% by weight of the end use composition. Conventional humectants may optionally be employed as additives in the present invention to assist in moisturizing skin when such end use compositions (i.e., emulsions) are topically applied. These are generally polyhydric alcohol type materials. Typical polyhydric alcohols include glycerol (i.e., glycerine or glycerin), propylene glycol, dipropylene glycol, polypropylene glycol (e.g., PPG-9), polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.0 to 15% by weight of the total weight of the compositions. Often, humectant makes up from 0.1 to 10%, and preferably, from 0.1 to 2.5% by weight (most preferably, from 0.2 to 1.5% by weight) of the total weight of the end use composition.

As to the end use compositions of the present invention, the same typically have from 1 to 35%, and preferably, from 2 to 30%, and most preferably, from 3 to 16% by weight total surfactant, based on total weight of the end use composition and including all ranges subsumed therein. In an embodiment of the invention, the end use composition comprises from 7 to 10% by weight total surfactant based on total weight of the end use composition and including all ranges subsumed therein.

The present invention is directed to hydratable concentrated surfactant composition that thickens and thus displays an increase in viscosity when mixed and diluted with water. In an embodiment of the invention, when the weight percent of zwitterionic surfactant to the weight percent of anionic surfactant exceeds 3:1 in the compositions, structuring agent (e.g., lauric acid) should be present at over 15% by weight of the total weight of surfactant in the compositions. Additionally, and in another embodiment of the invention, when the zwitterionic surfactant to anionic surfactant weight ratio is less than 1.5, structuring agent makes up 27% by weight or less of the total weight of surfactant in the compositions. In another embodiment, when the weight percent of zwitterionic surfactant to the weight percent of anionic surfactant exceeds 3:1 in the compositions, structuring agent (e.g., lauric acid) should be present at over 15% by weight of the total weight of surfactant in the compositions and when the zwitterionic surfactant to anionic surfactant weight ratio is less than 1.5, structuring agent makes up 27% by weight or less than the total weight of surfactant in the compositions.

When making hydratable composition of the present invention, the desired ingredients may be mixed with conventional apparatus under moderate shear and atmospheric conditions, with temperature being from 35 to 80°C. Water is added to the hydratable composition to produce the end use composition. Moderate shear such as shaking (or stirring) in a container will yield the end use composition in less than 5 minutes, preferably in less than 3 minutes, and most preferably, in less than 2 minutes. In an embodiment of the invention, end use composition is made in less than 1 minute, even preferably, less than 30 seconds. The end use composition will typically comprise 65 to 95%, and preferably, from 65 to 90%, and most preferably, from 65 to 88% by weight water based on total weight of the end use composition. In an embodiment of the invention, water makes up from 68 to 85% by weight of the end use composition, and in another embodiment, 72 to 80% by weight of the end use composition.

Accordingly, the present invention relates in a further aspect to a method to prepare an end use composition, the method comprising the step of diluting a hydratable composition of the present invention with water. Preferably, the composition is diluted at a composition to water weight ratio from 1:1 to 1:10. The hydratable concentrated surfactant composition has a viscosity from 25 to 10,000 cps and upon dilution the viscosity increases resulting in an end use composition having a viscosity from 1,000 to 20,000 cps, The viscosity is measured with a Discovery HR-2 Rheometer using sand blasted plates with a 100 micron gap and a shear rate of 4-15 s-1 and at a temperature of 25 °C.

The packaging for the compositions typically is not limited as long as hydratable composition can be hydrated and end use composition can be made upon the addition of water. In an embodiment on the invention, the hydratable composition is sold in a pouch (including polyvinyl alcohol sachet) or cartridge that is associated with and inserted in a bottle or canister. The bottle or canister is one which is filled with water and allows for the release of the hydratable composition into the same for mixing with water. Typically, the bottle or canister has a cap with a pump that opens the sachet or canister to release the hydratable composition into the water to make end use composition. Such a hydratable composition unexpectedly yields an end use composition, such as a body wash, with desirable characteristics appreciated by consumers. The packaging allows for infinite numbers of refilling to invariably reduce plastic waste in the environment.

The hydratable concentrated composition is preferably packaged in a refill packaging. Preferably, the hydratable concentrated composition is used as a refill-composition. Packaging that includes post consumer resin is often preferred.

The Examples provided are to facilitate an understanding of the invention. They are not intended to limit the scope of the claims.

### Example I

Illustrative hydratable concentrated surfactant compositions consistent with this invention and assessed in Example II were prepared by combining the ingredients identified below.

Each Sample of this Example, as set forth in the Tables, were made by conventional means, and therefore, by mixing ingredients with moderate shear under atmospheric conditions at a temperature from about 35 to 75°C. Versathix^{™} is PEG 150 pentaerythritol tetrastearate.

### Sample I

| **Ingredient** | **Weight Percent** |
|---|---|
| DI Water | 61.77 |
| Cocamidopropyl Betaine | 14.13 |
| Sodium Hydroxide | 0.68 |
| Lauric Acid | 4.62 |
| Sodium Lauroyl Glycinate | 0.00 |
| Sodium Lauroyl Taurate | 10.87 |
| Glycerin | 0.95 |
| Chelator | 0.05 |
| Preservative | 0.61 |
| Sodium Chloride | 3.0 |
| Versathix^{™} | 0.32 |
| Fragrance | 3.0 |
| Total | 100 |

### Sample II

| **Ingredient** | **Weight Percent** |
|---|---|
| DI Water | 61.77 |
| Cocamidopropyl Betaine | 14.13 |
| Sodium Hydroxide | 0.68 |
| Lauric Acid | 4.62 |
| Sodium Lauroyl Glycinate | 2.0 |
| Sodium Lauroyl Taurate | 8.87 |
| Glycerin | 0.95 |
| Chelator | 0.05 |
| Preservative | 0.61 |
| Sodium Chloride | 3.0 |
| Versathix | 0.32 |
| Fragrance | 3.0 |
| Total | 100 |

### Example II

The compositions (i.e., end use/diluted compositions) in the Table were made by hydrating the hydratable concentrated surfactant compositions (from Example I). The hydratable compositions were diluted (water to composition) at a weight ratio of 2 to 1. For the avoidance of doubt, "Concentrate Viscosity" means the viscosity of hydratable composition and "Dilute Viscosity" means the viscosity of the end use wash composition made, both in centipoise (cps). Water and hydratable composition were combined in a vessel and were agitated with mild shaking. In less than one (1) minute, desired end use wash compositions (transparent/isotropic and homogeneous) were unexpectedly obtained with increased viscosities.

**Table**

| **Sample** | **CAPB** | **Glycinate** | **Taurate** | **Lauric Acid** | **NaCl** | **Concentrate Viscosity** | **Dilute Viscosity** |
|---|---|---|---|---|---|---|---|
| 2 | 21.2% | 2% | 8.8% | 4.6% | 3% | 1830 | 7742 |
| 1 | 21.2% | 0% | 10.8% | 4.6% | 3% | 1459 | 6845 |

| | | | | | | | |
|---|---|---|---|---|---|---|---|
| CAPB-cocamidopropyl betaine Glycinate-sodium Lauroyl Glycinate Taurate- sodium methyl lauroyl taurate Versathix- PEG 150 pentaerythritol tetrastearate | | | | | | | |

As can be seen from the data provided, the end use compositions made according to this invention where prepared in less than one (1) minute of agitation and they surprisingly thickened (increased in viscosity) when combined with water. The hydratable compositions were hazy (lamellar) and the end use compositions were surprisingly thicker yet transparent (isotropic) after visual inspection. The transformation from a lamellar phase to an isotropic phase was also confirmed when the compositions were placed on a glass slide and assessed in an optical microscope fitted with cross-polarizers. The end use compositions did not display a birefringent pattern.

## Claims

1. A cleansing composition comprising:
a) an anionic surfactant;
b) an amphoteric and/or zwitterionic surfactant;
c) a C₆ -C₁₄ acid, C₆ -C₁₄ alcohol and/or C₆ -C₁₄ amide;
d) from 30 to 85% by weight water, forming a lamellar precursor composition, and
e) structuring agent,
wherein less than 0.0075% by weight of the anionic surfactant is isethionate,
wherein the anionic surfactant is from 35 to 100% by weight taurate, preferably 100% by weight taurate, and further
wherein the cleansing composition comprises less than 1% by weight sulfate, preferably no sulfate,
wherein the cleansing composition comprises inorganic salt, and
wherein the pH is from 4.9 to 6.0.

2. The composition according to claim 1, wherein the composition is substantially free of oil and sulphate, and preferably, is free of oil and sulphate, the precursor composition comprising 3 to 20%, preferably, 6 to 15% or more preferably 7 to 12%, by weight anionic surfactant and 6 to 25%, preferably 12 to 22%, by weight zwitterionic surfactant.

3. The composition according to any one of the preceding claims, wherein the anionic surfactant is a taurate or a mixture of taurate and glycinate.

4. The composition according to claim 3 wherein the anionic surfactant comprises an acyl taurate.

5. The composition according to claim 3 or 4, wherein the surfactant comprises an acyl glycinate.

6. The composition according to any one of the preceding claims wherein the composition further comprises a resorcinol, niacinamide, 12-hydroxystearic acid or a mixture thereof.

7. The composition according to any one of the preceding claims, wherein the structuring agent comprises PEG150 pentaerythritol tetrastearate.

8. The composition according to any one of the preceding claims, wherein the composition comprises sodium benzoate.

9. The composition according to any one of the preceding claims, wherein the composition comprises no isethionate and no sulphate and comprises zwitterionic surfactant comprising a betaine.

10. A method for making an end use cleansing composition comprising from more than 65% to 95% by weight water, the method comprising the steps of:
a) combining a composition according to any one of claims 1 to 9 with water to produce a water and hydratable composition mix;
b) agitating and/or shearing the mix;
to provide an end-use cleansing composition,
wherein the end-use composition has a viscosity which is greater than the viscosity of the hydratable precursor cleansing composition.

11. A method according to claim 10, wherein the end use composition has a pH less than 7 and a viscosity from 1,000 to 20,000 cps (1,000 to 20,000 mPa-s), and preferably, from 3,000 to 12,000 cps (3,000 to 12,000 mPa-s), as measured at 25 °C and using a rheometer using a 100 micron gap and a shear rate of 4-15 s⁻¹.

12. Use of a composition according to any one of claims 1 to 9 for cleansing skin upon dilution of said composition in water.

## Patentansprüche

1. Reinigungszusammensetzung, umfassend:
a) ein anionisches Tensid;
b) ein amphoteres und/oder zwitterionisches Tensid;
c) eine C₆-C₁₄-Säure, einen C₆-C₁₄-Alkohol und/oder ein C₆-C₁₄-Amid;
d) 30 bis 85 Gewichts-% Wasser, wodurch eine lamellare Vorläuferzusammensetzung gebildet wird, und
e) ein Strukturierungsmittel,
wobei weniger als 0,0075 Gewichts-% des anionischen Tensids Isethionat sind, wobei das anionische Tensid zu 35 bis 100 Gewichts-% aus Taurat, bevorzugt zu 100 Gewichts-% aus Taurat, besteht und
wobei die Reinigungszusammensetzung ferner weniger als 1 Gewichst-% Sulfat, bevorzugt kein Sulfat umfasst,
wobei die Reinigungszusammensetzung anorganisches Salz umfasst und der pH-Wert 4,9 bis 6,0 beträgt.

2. Zusammensetzung nach Anspruch 1, wobei die Zusammensetzung im Wesentlichen frei von Öl und Sulfat ist und bevorzugt frei von Öl und Sulfat ist, wobei die Vorläuferzusammensetzung 3 bis 20%, bevorzugt 6 bis 15% oder bevorzugter 7 bis 12%, bezogen auf das Gewicht des anionischen Tensids, und 6 bis 25%, bevorzugt 12 bis 22%, bezogen auf das Gewicht des zwitterionischen Tensids, umfasst.

3. Zusammensetzung nach irgendeinem der vorhergehenden Ansprüche, wobei das anionische Tensid ein Taurat oder eine Mischung aus Taurat und Glycinat ist.

4. Zusammensetzung nach Anspruch 3, wobei das anionische Tensid ein Acyltaurat umfasst.

5. Zusammensetzung nach Anspruch 3 oder 4, wobei das Tensid ein Acylglycinat umfasst.

6. Zusammensetzung nach irgendeinem der vorhergehenden Ansprüche, wobei die Zusammensetzung ferner ein Resorcin, Niacinamid, 12-Hydroxystearinsäure oder eine Mischung davon umfasst.

7. Zusammensetzung nach irgendeinem der vorhergehenden Ansprüche, wobei das Strukturierungsmittel PEG-150 Pentaerythrityltetrastearat umfasst.

8. Zusammensetzung nach irgendeinem der vorhergehenden Ansprüche, wobei die Zusammensetzung Natriumbenzoat umfasst.

9. Zusammensetzung nach irgendeinem der vorhergehenden Ansprüche, wobei die Zusammensetzung kein Isethionat und kein Sulfat umfasst und Betain einbeziehendes zwitterionisches Tensid umfasst.

10. Verfahren zur Herstellung einer Reinigungszusammensetzung für den Endverbrauch, umfassend mehr als 65 bis 95 Gewichts-% Wasser, wobei das Verfahren die Schritte umfasst:
a) Kombinieren einer Zusammensetzung nach irgendeinem der Ansprüche 1 bis 9 mit Wasser, um eine Mischung aus Wasser und hydratisierbarer Zusammensetzung herzustellen;
b) Rühren und/oder Scheren der Mischung;
um eine Reinigungszusammensetzung für den Endgebrauch herzustellen,
wobei die Zusammensetzung für den Endgebrauch eine Viskosität aufweist, die größer als die Viskosität der hydratisierbaren Vorläufer-Reinigungszusammensetzung ist.

11. Verfahren nach Anspruch 10, wobei die Zusammensetzung für den Endgebrauch einen pH-Wert von weniger als 7 und eine Viskosität von 1.000 bis 20.000 cps (1.000 bis 20.000 mPa.s) und bevorzugt 3.000 bis 12.000 cps (3.000 bis 12.000 mPa.s), gemessen bei 25°C und unter Verwendung eines Rheometers mit einem 100 Mikrometer-Spalt und bei einer Scherrate von 4-15 s⁻¹, aufweist.

12. Verwendung einer Zusammensetzung nach irgendeinem der Ansprüche 1 bis 9 zum Reinigen der Haut bei Verdünnung der Zusammensetzung in Wasser.

## Revendications

1. Composition nettoyante comprenant :
a) un tensioactif anionique ;
b) un tensioactif amphotère et/ou zwittérionique ;
c) un acide en C₆ à C₁₄, un alcool en C₆ à C₁₄ et/ou un amide en C₆ à C₁₄ ;
d) 30 à 85 % en poids d'eau, formant une composition précurseur lamellaire, et
e) un agent structurant,
dans laquelle moins de 0,0075 % en poids du tensioactif anionique consiste en un iséthionate,
dans laquelle le tensioactif anionique consiste en 35 à 100 % en poids de taurate, de préférence 100 % en poids de taurate, et en outre
laquelle composition nettoyante comprend moins de 1 % en poids de sulfate, de préférence ne comprend pas de sulfate,
laquelle composition nettoyante comprend un sel inorganique, et
dans laquelle le pH est de 4,9 à 6,0.

2. Composition selon la revendication 1, laquelle composition est pratiquement exempte d'huile et de sulfate, et de préférence est exempte d'huile et de sulfate, la composition précurseur comprenant 3 à 20 %, de préférence 6 à 15 % ou mieux encore 7 à 12 % en poids de tensioactif anionique et 6 à 25 %, de préférence 12 à 22 % en poids de tensioactif zwittérionique.

3. Composition selon l'une quelconque des revendications précédentes, dans laquelle le tensioactif anionique est un taurate ou un mélange de taurate et de glycinate.

4. Composition selon la revendication 3, dans laquelle le tensioactif anionique comprend un acyltaurate.

5. Composition selon la revendication 3 ou 4, dans laquelle le tensioactif comprend un acylglycinate.

6. Composition selon l'une quelconque des revendications précédentes, laquelle composition comprend en outre un résorcinol, du niacinamide, de l'acide 12-hydroxystéarique ou un mélange de ceux-ci.

7. Composition selon l'une quelconque des revendications précédentes, dans laquelle l'agent structurant comprend du tétrastéarate de pentaérythritol PEG150.

8. Composition selon l'une quelconque des revendications précédentes, laquelle composition comprend du benzoate de sodium.

9. Composition selon l'une quelconque des revendications précédentes, laquelle composition ne comprend pas d'iséthionate et pas de sulfate et comprend un tensioactif zwittérionique comprenant une bétaïne.

10. Procédé pour préparer une composition nettoyante à usage final comprenant de plus de 65 % à 95 % en poids d'eau, le procédé comprenant les étapes de :
a) combinaison d'une composition selon l'une quelconque des revendications 1 à 9 avec de l'eau pour produire un mélange de composition hydratable et d'eau ;
b) agitation et/ou secouage du mélange ;
pour former une composition nettoyante à usage final,
dans lequel la composition à usage final a une viscosité qui est supérieure à la viscosité de la composition nettoyante précurseur hydratable.

11. Procédé selon la revendication 10, dans lequel la composition à usage final a un pH inférieur à 7 et une viscosité de 1 000 à 20 000 cps (1 000 à 20 000 mPa-s) et de préférence de 3 000 à 12 000 cps (3 000 à 12 000 mPa-s), telle que mesurée à 25°C et au moyen d'un rhéomètre utilisant un espace de 100 micromètres et une vitesse de cisaillement de 4 à 15 s⁻¹.

12. Utilisation d'une composition selon l'une quelconque des revendications 1 à 9 pour nettoyer la peau après dilution de ladite composition dans de l'eau.
